# EUROPEAN PATENT APPLICATION

(11) **EP 0 964 037 A1**
(43) Date of publication of application: **15.12.1999**
(21) Application number: 98905701.3
(22) Date of filing: 27.02.1998
(51) Int. Cl.: C09D 127/12, C09D 201/00, C09D 167/00, C09D 123/08, C09D 133/04, A61L 29/00, A61L 33/00, A61M 5/14, A61M 5/32

(54) **COATING COMPOSITION, COATED OBJECT, AND COATING METHOD**

(30) Priority: 28.02.1997 JP 4638597
(71) Applicant: Mori, Yuichi, Yokohama-shi, Kanagawa 236-0045 (JP)
(72) Inventor: MORI, Yuichi, Yokohama-shi Kanagawa 236-0045 (JP); YOSHIOKA, Hiroshi, Hadano-shi Kanagawa 257-0026 (JP); KUBOTA, Sunao, Tokyo 186-0002 (JP)
(74) Representative: Mackenzie, Andrew Bryan
(86) International application number: JP9800829
(87) International publication number: WO9838258

(57) **Abstract**

Ships, outdoor communication equipment (for prevention of ice and snow accretion), devices for body fluid contact or for insertion into living body tissue, etc., are coated by using a coating composition comprising at least, particulates comprising a fluorine-containing polymer and a non-fluorine-containing polymer. There is formed a coating which is excellent in its lubricating property (friction-reducing property) and in its adhesion to the device or in its coating strength.

## Description

### Technical Field

The present invention relates to a composition for coating capable of forming, on surfaces of various kinds of base materials or substrates, a coating which is not only excellent in the lubricating property (friction-reducing property) thereof, but also is excellent in the adhesion property, or the coating strength thereof to the base material, etc., to a coated product formed from the coating composition, and a coating method using the coating composition.

It is very easy to impart to the coating composition according to the present invention an effect of preventing the adhesion of at least one component of a body fluid (in the present invention, this term is used so as to include a "secreted fluid" discharged to the outside of a body such as sweat, saliva, tears and urine), as desired. Accordingly, when the coating composition according to the present invention is applied to the surface of a device or equipment to be put in contact with the body fluid or to be inserted into the tissue of a living body (e.g., a medical device such as an injection needle), it is possible to impart a lubricating property to such a surface together with an effect of preventing the adhesion of the body fluid component.

The coating composition according to the present invention in an embodiment to which the lubricating property as well as the effect of preventing the adhesion of the body fluid component is imparted as described above, may be particularly suitably applicable to the coating of a medical device which is intended to penetrate into the tissue of a living body such as skin or to be inserted into a body cavity such as alimentary canal, or lumen such as blood vessel, so that it can perform various kinds of important functions in medical service such as introduction of medicine, removal of effusion, pus and waste, monitoring of signals from a living body, or maintenance of the patency of the lumen. Examples of such a medical device may include injection needle, indwelling needle, tube, catheter, cannula, guide wire, stent, etc.

The coating composition according to the present invention in an embodiment to which the lubricating property as well as the effect of preventing the adhesion of the body fluid component is imparted, can suitably impart to these medical devices not only a property of suppressing the adhesion of the body fluid component (or living body component) such as blood clot or thrombus, but also a surface lubricating property which enables smooth insertion of the device into the living body tissue through penetration, or smooth introduction into the body cavity such as alimentary canal or a narrow lumen such as blood vessel.

### Background Art

Herein, there is first described the background art relating to the coating composition according to the present invention in an embodiment to which the lubricating property as well as the effect of preventing the adhesion of the body fluid component is imparted, although such a coating composition is also applicable without particular limitation to other fields (such as field of the prevention of ice accretion on ships or vessels, etc., and field of outdoor communication equipment in an area of heavy snowfall) wherein there is demanded the formation of a coating on the surfaces of various kinds of base materials or substrates which is excellent in the lubricating property and adhesion property (or coating strength) to the base material, etc.

In general, blood has an important property such that when a blood vessel is damaged, and the blood flows out of the blood vessel externally, the discharged blood is immediately coagulated and produces a blood clot (or thrombus) so as to prevent excessive bleeding. On the other hand, however, with respect to the medical devices to be used for the insertion thereof into the blood vessel, such a property of the blood causes a serious problem such that a blood clot is formed on the surface of the medical device material, and the function of the medical device is lost on the basis of the blood clot.

The mechanism (system) by which the blood clot is formed on the surface of a material has not been fully elucidated, but through a large number of previous in vitro experiments, animal experiments, clinical experiments, etc., the correlation between the physical property of a material and the formation of a blood clot has been gradually clarified although such a correlation is semi-empirical. According to such a semi-empirical mechanism, it is considered that the blood clot formation is triggered by the adhesion of some blood components such as a certain kind of plasma protein (fibrinogen), and platelets onto the material surface and the activation of these components. From such a viewpoint, there have been practiced some approaches such that the adhesion of blood components such as plasma protein and platelets onto the material surface is intended to be suppressed as completely as possible for the purpose of suppressing the blood clot formation.

As one of these approaches, there has bean reported an attempt wherein the adhesion of the body fluid component to a material is suppressed by imparting a micro-heterogeneous structure to the surface of the material (Yoji Imai, Antithrombogenic Materials - Status Quo and Prospects, Artificial Organs, 12, 963, 1983). Particularly, it is reported that an excellent antithrombogenic property can be obtained when the microheterogeneous structure is a microphase-separated structure formed from a hydrophobic domain and a hydrophilic domain. For example, it is reported that a catheter was prepared from a ethylene-acrylic acid (salt) copolymer comprising ethylene as a hydrophobic portion and acrylic acid (salt) as a hydrophilic portion, and is subjected to animal experiments using a dogs and clinical experiments (human), and there were investigated the resultant frequencies of the occurrence (or incidence) of blood clot formation to the catheter and the phlebitis, etc., which is considered to be attributable to the thus formed blood clot. In view of the frequency of the occurrence of the phlebitis, it is reported that the antithrombogenic property of the ethylene-acrylic acid (salt) copolymer is particularly good as compared with those in the catheters comprising polyethylene, polypropylene, polyvinyl chloride, polytetrafluoroethylene, etc., (P. N. Sawyer, at al., Experimental and Clinical Evaluation of a New Catheter Material, Trans. Amer. Soc. Artif. Int. Organs, 22, 527, 1976).

In the case of the above-mentioned ethylene-acrylic acid (salt) copolymer, it is considered that the ethylene unit of the hydrophobic portion and the acrylic acid (salt) unit of the hydrophilic portion are microheterogeneous so as to form a heterogeneous structure which suppresses the adhesion of the blood component and exhibits an antithrombogenic property. In contrast, it is considered the surfaces of the other materials of polyethylene, polypropylene, polyvinyl chloride, polytetrafluoroethylene, etc., have a hydrophobic homogeneous structure, and they cannot suppress the adhesion of the blood component.

On the other hand, in contrast to the above-mentioned homogeneous hydrophobic surface, the influence or a homogeneous hydrophilic surface on blood has also been investigated. For example, it is reported that a surface grafted with polyhydroxyethyl methacrylate having a homogeneous hydrophilic structure damages the blood components such as platelets, since the strong polar groups thereof activate the blood coagulation factor and a complement (Ratner BD, et al., Blood Compatibility-Water Content Relationships for Radiation-Grafted Hydrogels, J. Polym. Sci.: Polym. Symp., 66, 363, 1979).

As described above, when the hydrophobic portion and the hydrophilic portion form a micro-heterogeneous structure, it is considered that such a structure suppresses the adhesion and activation of the body fluid components and as a result, exhibits an antithrombogenic property as compared with in the homogeneous structure comprising either a hydrophobic portion, or a hydrophilic portion alone.

On the other hand, in current medical services, there are frequently used injection needles, indwelling needles, inner tubes, catheters, cannulas, guide wires, stents, etc., which penetrate into the tissue of a living body such as skin and into a body cavity such as the alimentary canal, the trachea or bronchi, or blood vessels, so that they can perform the introduction of medicine, removal of effusion, pus and waste, monitoring of signals from a living body, or maintenance of the lumen, etc.

When these medical devices are used, it is necessary to suppress the adhesion of body fluid components such as blood clots to the material surface as described above and, further, the lubricating property, i.e., friction-reducing property of the material surface becomes an important property. For example, in the case of the injection needle or indwelling needle which is to be used so as to penetrate the skin, etc., when the lubricating property of material surface is poor, the inserting manipulation is difficult, and simultaneously it causes pain for the patient.

In a case where the above-mentioned medical device is inserted into a complicatedly bent or curved body cavity or blood vessel, when the lubricating property of the material surface thereof is poor, the inserting manipulation per se becomes difficult, and it causes pain for the patient, and further, it can damage the tissue in the body cavity. AS a result, the use of the medical device for the purpose of treatment or diagnosis can cause a disorder or lesion such as new infection and accretion. In addition, when these disorders occur in the blood vessel, the damage caused by the disorder increases the possibility of the induction of further blood clot formations.

Heretofore, for the purpose of imparting the lubricating property to the surfaces of these medical devices, there have been practiced a method wherein a device surface is coated with a polymer having a friction-reducing surface such as fluorine-containing polymer and hydrophilic polymer, and a method wherein the device surface is coated with a lubricating liquid such as silicone oil. However, it is actually difficult to coat the surfaces or these medical devices comprising a metal or a polymer material rich in flexibility with a fluorine-containing polymer or hydrophilic polymer so as to provide a sufficient strength and adhesion therebetween which is enough to prevent the breakage or peeling thereof due to the manipulation of inserting the device into a living body.

In addition to the above-mentioned problem, when the medical device is simply coated with a lubricating liquid such as silicone oil, the lubricating liquid inevitably enters the interior of the body, whereby the lubricating liquid can cause various problems.

Further, as described above, it is difficult for both of the homogeneous hydrophobic surface of a fluorine-containing polymer, etc., and the homogeneous hydrophilic surface comprising a hydrophilic polymer, to prevent the adhesion of body fluid components, the activation thereof and blood clot formation. Accordingly, there has been eagerly desired the development of a surface which is not only capable of effectively preventing the adhesion of the body fluid component, the activation thereof and the blood clot formation, etc., but also is capable of imparting a lubricating property. However, in the prior art, there has never been obtained a coating composition which is capable of imparting a satisfactory characteristic to a medical device.

In the fields other than the above-mentioned medical device (such as field of the prevention of ice accretion on ships, and field of outdoor communication equipment in an area of heavy snowfall), there has eagerly been desired the formation of a coating on various kinds of base materials and substrates which is excellent in lubricating property and adhesion thereof (or coating strength) to the base material, etc. However, in the prior art, there has never been obtained a coating composition which is capable of imparting satisfactory lubricating and adhesion (or coating strength) properties to the surfaces of the various kinds of base materials and substrates.

An object of the present invention is to solve the above-mentioned problems encountered in the prior art, and to provide a coating composition which is capable of imparting satisfactory lubricating and adhesion (or coating strength) properties to the surfaces of the various kinds of base materials and substrates.

Another object of the present invention is to provide a coating composition which is capable of imparting both of a lubricating property and a resistance to the adhesion of a body fluid component such as antithrombogenic property.

A further object of the present invention is to provide a coating method which is capable of coating the surfaces of various kinds of base materials and substrates by using the above-mentioned coating composition.

A further object of the present invention is to provide a coated product having a coating layer which is excellent in both of the lubricating property and adhesion property (or coating strength).

A further object of the present invention is to provide a coated product having a coating layer which is excellent in both of the lubricating property and the resistance to the adhesion or a body fluid component. Disclosure of Invention

As a result of earnest study, the present inventors have found that it is extremely effective in achieving the above-mentioned objects to form a coating layer covering at least a part of a base material or substrate by use of a coating composition comprising fluorine-containing polymer particulates in combination with another non-fluorine-containing polymer, thereby to realize both an excellent lubricating property and an excellent adhesion property (or coating strength) on the basis of the synergistic effect of these components.

The coating composition according to the present invention is based on the above-mentioned discovery, and comprises: at least, particulates comprising a fluorine-containing polymer; and a non-fluorine-containing polymer.

The present invention also provides a coating method, comprising:
attaching a coating composition comprising at least particulates comprising a fluorine-containing polymer, and a non-fluorine-containing polymer, to the surface of a material to be coated by powder coating; and
heating the non-fluorine-containing polymer to a temperature higher than the softening point thereof, thereby to form a coating layer comprising the coating composition on the surface of the material to be coated.

The present invention further provides a coating method, comprising:
dispersing a coating composition comprising at least particulates comprising a fluorine-containing polymer, and a non-fluorine-containing polymer, in a solvent capable of dissolving the non-fluorine-containing polymer thereby to prepare a coating liquid; and
applying the coating liquid onto the surface of a material to be coated, thereby to form a coating layer comprising the coating composition on the surface of the material to be coated.

According to the present inventors' knowledge, in the step of coating the surface of a material to be coated with the above-mentioned coating composition, it is presumed that even when the above-mentioned non-fluorine-containing polymer is heated, fused or dissolved, the fluorine-containing polymer particulates having a low coefficient of friction in the coating composition have a tendency (based on their characteristic as fluorine-containing polymer) such that they are not fused or dissolved but they retain their particulate shapes, and therefore they are exposed to the surface of the coating layer which has been formed on the surface of the material to be coated so as to impart the lubricating property to the surface. In other words, it is presumed that in the coating layer to be formed on the material to be coated by using the composition according to the present invention, the lubricating property and micro convexities and concavities structure based on the surface-exposed fluorine-containing polymer particulates and the above-mentioned non-fluorine-containing polymer function synergistically.

As a result of further study by the present inventors based on the above-mentioned discovery, it has been found that it is very effective in achieving the above-mentioned objects to form a coating layer covering a part of a material to be coated which is to be in contact with body fluid or to be inserted into a living body by using a coating composition comprising particulates comprising a fluorine-containing polymer, and a copolymer comprising a hydrophobic monomer and a hydrophilic monomer, thereby to realize both an excellent non-adhesion property to the body fluid component and a lubricating property the basis of the synergistic effect of the physical and chemical functions of these components.

The coating composition according to the present invention (in an embodiment to which a non-adhesion property to body fluid component has also been imparted) is based on the above discovery, and comprises: at least, particulates comprising a fluorine-containing polymer; and a copolymer comprising a hydrophobic monomer and a hydrophilic monomer.

The present invention also provides a coated product, comprising a material to be coated and a coating layer covering at least a portion of the material to be coated to be in contact with body fluid; wherein the coating layer comprises at least particulates comprising a fluorine-containing polymer; and a copolymer comprising a hydrophobic monomer and a hydrophilic monomer.

According to the present inventors' knowledge, it is presumed that the copolymer of hydrophobic monomer/hydrophilic monomer constituting the coating composition according to the present invention in the embodiment to which the above-mentioned non-adhesion property to the body fluid component can also be imparted, forms a micro-heterogeneous structure due to a phase-separation phenomenon while forming a continuous layer in the step of coating the surface of the material to be coated with the coating composition (e.g., a step of heating to a temperature higher than the softening point of the copolymer or a step of effecting solvent coating). Accordingly, it is presumed that, on the basis of the above-mentioned micro-heterogeneous structure of the coating which has been imparted to the surface of the material to be coated by the coating composition according to the present invention, the adhesion and activation of the body fluid component, and the blood clot formation are effectively prevented.

According to the present inventors' knowledge, it is presumed that in the coating layer to be formed on the material to be coated by the composition according to the present invention, the lubricating property/micro convexities and concavities structure based on the thus surface-exposed fluorine-containing polymer particulates and the micro-heterogeneous structure of the above-mentioned copolymer function synergistically. In other words, in the present invention (in the embodiment to which the non-adhesion property to the body fluid component can also be imparted), it is presumed that the physical and chemical effects respectively based on the above-mentioned fluorine-containing polymer particulates and the copolymer of hydrophobic monomer/hydrophilic monomer are combined, and the entirety of such a combination exhibits excellent non-adhesion and lubricating properties.

In such an embodiment of the present invention, the above-mentioned effect is particularly remarkable when the above-mentioned fluorine-containing polymer is polytetrafluoroethylene. According to the present inventors' experiments, it has unexpectedly been found that the fluorine-containing polymer (particularly, polytetrafluoroethylene) particulates which have been exposed to the coating surface provided by the present invention do not lower the antithrombogenic property of the coating surface but rather enhance the antithrombogenic property thereof. According to the present inventors' knowledge, it is presumed that this phenomenon in the coating layer according to the present invention is based on the results of the above-mentioned suitable combination of the phase-separation phenomenon of the hydrophobic portion and hydrophilic portion (Yoji Imai, Antithrombogenic Materials - Status Quo and Prospects, Artificial Organs, 12, 963, 1983), etc., and the provision of the uneven structure to surface by the particulates.

On the other hand, according to the present inventors' knowledge, it is presumed that the hydrophilic portion in the copolymer in the coating composition according to the present invention in the above-mentioned embodiment has a high polarity, and has a function of remarkably increasing the adhesive property to the surface of the material to be coated such as metal. According to the present inventors' experiments, it is found that the effect of such an increase in the adhesive property becomes remarkable when the hydrophilic monomer is acrylic acid (salt). Brief Description of Drawings
Fig. 1 is a schematic sectional view showing an embodiment of the coating composition (physically mixed state) according to the present invention.
Fig. 2 is schematic sectional view showing embodiment other than the coating composition (composite particulate state) according to the present invention.
Fig. 3 is a schematic sectional view showing an embodiment of the coated product comprising a material to be coated, and a coating layer formed thereon on the basis of the coating composition according to the present invention.
Fig. 4 is an image showing an example of the state of a coating layer at the tip of a needle coated with a PTFE-EAA coating prepared in an Example appearing hereinafter.
Fig. 5 is an image showing another example of the state of a coating layer at the tip of a needle coated with a PTFE-EAA coating prepared in an Example appearing hereinafter.
Fig. 6 is an image showing an example of the state of a coating layer at the tip of a needle coated with a PET-EAA coating prepared in an Example appearing hereinafter.
Fig. 7 is an image showing another example of the state of a coating layer at the tip of a needle coated with a PET-EAA coating prepared in an Example appearing hereinafter.
Fig. 8 is an image showing an example of the state of a coating layer at the outside surface of a stent coated with a PTFE-EAA coating prepared in an Example appearing hereinafter.
Fig. 9 is an image showing another example of the state of a coating layer at the inside surface of a stent coated with a PTFE-EAA coating prepared in an Example appearing hereinafter.

### Best Mode for Carrying out the Invention

Hereinbelow, the present invention will be described in detail with reference to the accompanying drawings as desired. In the following description, "%" and "part(s)" representing a quantitative proportion or ratio are those based on weight, unless otherwise noted specifically.

### (Coating composition)

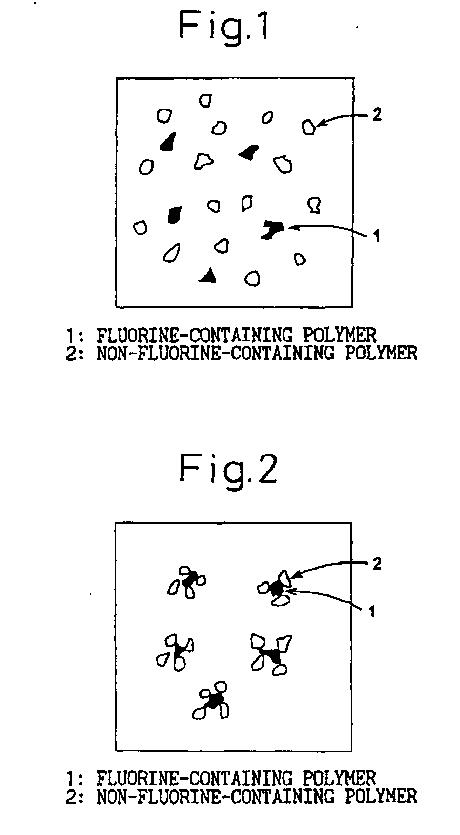

The coating composition according to the present invention is a composition which comprises, at least, particulates comprising a fluorine-containing polymer, and a non-fluorine-containing polymer. In the present invention, the shapes of the particulates and the non-fluorine-containing polymer are not particularly limited as long as they are co-present so that they can form a coating layer on a material to be coated. For example, in the present invention, as shown in the schematic sectional view of Fig. 1, particulates 1 comprising the fluorine-containing polymer and a non-fluorine-containing polymer 2 may be mixed with each other both in the form of powder, so as to constitute the coating composition. Alternatively, the coating composition may also comprise particulates 1 comprising the fluorine-containing polymer, and a non-fluorine-containing polymer 2 disposed on the surfaces of the particulates 1 so as to form composite particles. In Fig. 2, the non-fluorine-containing polymer 2 is disposed on the particulate 1 in the form of a discontinuous state, but the non-fluorine-containing polymer 2 may also be disposed on the particulate 1 in the form of a substantially continuous layer (i.e., in the farm or a microcapsule-like state).

### (Fluorine-containing polymer)

The kind, the monomer composition, the molecular weight, etc., of the fluorine-containing polymer usable in the present invention are not particularly limited. In view of the possible provision of a suitable lubricating property to a material to be coated, the average fluorine content in one molecule of the polymer, i.e., [19 × (number of average fluorine atoms contained in one fluorine-containing polymer molecule)]/(weigh-average molecular weight of fluorine-containing polymer) may preferably be 40% or more, more preferably about 49 - 76% (particularly, about 60 - 76%).

Similarly, in view of the possible provision of a suitable lubricating property to the material to be coated, the critical surface tension (20°C) of the fluorine-containing polymer may preferably be about 25 dyn/cm or less, more preferably about 22 dyn/cm or less (particularly about 19 dyn/cm or less).

In the present invention, in view of the lubricating property, it is preferred to use polytetrafluoroethylene (PTFE), polytrifluoroethylene, polyvinylidene fluoride, etc., as the above-mentioned fluorine-containing polymer, and particularly preferably polytetrafluoroethylene among them.

In view of the size of minute unevenness to be imparted to the coating formed from the coating composition according to the present invention, the average particle size of the fluorine-containing polymer particulates may preferably be about 100 µm or less, more preferably about 50 µm or less (particularly about 20 µm or less).

The shape of the fluorine-containing polymer particulate are not particularly limited, but it is possible to utilize various kinds of shapes such as spherical, non-spherical (cylindrical shape, plate-like shape, indeterminate shape, etc.) depending on the process for producing such particulates.

### (Non-fluorine-containing polymer)

The non-fluorine-containing polymer constituting the coating composition according to the present invention together with the above-mentioned particulates comprising the fluorine-containing polymer is a polymer which does not substantially contain fluorine in the molecule thereof. The kind, the monomer composition, the molecular weight, etc., of the non-fluorine-containing polymer usable in the present invention are not particularly limited, as long it is a polymer which does not substantially contain fluorine in the molecule thereof.

In view of the possible provision of a suitable adhesion property or coating strength to the material to be coated, the average fluorine content in one molecule of the polymer, i.e., [19 × (number of average fluorine atoms contained in one fluorine-containing polymer molecule)]/(weight average molecular weight of fluorine-containing polymer) may preferably be about 10% or less, and more preferably about 1% or less (particularly, about 0.1% or less).

Similarly, in view of the possible provision of a suitable adhesion property or coating strength to the material to be coated, the fluorine-containing polymer may preferably be distributed in the surface layer portion of the coating. Accordingly, the critical surface tension of the non-fluorine-containing polymer may preferably be larger than that of the fluorine-containing polymer (i.e., the non-fluorine-containing polymer may preferably be less liable to be wetted with the fluorine-containing polymer), and the critical surface tension (20°C) of the non-fluorine-containing polymer may preferably exceed about 25 dyn/cm, more preferably be about 30 dyn/cm or more (particularly about 40 dyn/cm or more).

In view of the ease of coating various kinds of material to be coated utilizing heat, the softening point (Sn) of the non-fluorine-containing polymer may preferably be lower than the softening point (Sf) of the fluorine-containing polymer. Further, the difference (Sf-Sn) between these softening points may preferably be 20°C or more, more preferably 50°C or more. With respect to the details of the method of measuring such a softening point, for example, literature (JIS K 7206 and ASTM D 1525) may be referred to.

In view of the ease of formation of the above-mentioned composite particulate having a shape similar to that of a microcapsule (e.g., composite particulate in the form as shown in Fig. 2) or composite particulate in the form of microcapsule, the above-mentioned non-fluorine-containing polymer may preferably be a polymer which can be disposed on the circumferences of the above-mentioned fluorine-containing polymer particulates by a known microencapsulation method. Specific examples of the known microencapsulation method usable for such a purpose may include: e.g., chemical microencapsulation methods (such as interfacial polymerization method, in-situ polymerization method, and orifice method), physicochemical microencapsulation methods (such as coacervation method, phase-separation method, and intersurface precipitation method), and physical/mechanical microencapsulation methods (such as spray-drying method) (with respect to the details of such various microencapsulation methods, e.g., Tamotsu Kondo/ Masumi Koishi "Microcapsules-New Edition", pages 1 - 72, 1987, Sankyo Shuppan may be referred to).

As an example of the polymer which can be disposed on the circumference of particulate by a microencapsulation method, it is possible to use either of a hydrophilic polymer and/or a hydrophobic polymer. Specific examples thereof may include polyester-type resins (such as polyethylene terephthalate, and polybutylene terephthalate), ethylene-acrylic acid copolymer (EAA), etc.

### (Copolymer or hydrophobic monomer/hydrophilic monomer)

In an embodiment wherein an effect of preventing the adhesion of the body fluid component is also imparted to the coating composition according to the present invention, it is preferred to use a polymer comprising a hydrophobic monomer and a hydrophilic monomer as the above-mentioned non-fluorine-containing polymer. Herein, "hydrophilic monomer" refers a monomer containing at least one hydrophilic group in the molecule thereof. The hydrophilic monomer may also have two or more (number) of, and/or two or more kinds of hydrophilic groups in the molecule thereof, as desired. As the hydrophilic groups usable may include functional groups such as -OH, -C-O-C- (ether group), -CO- (carbonyl group), -COOH (or salt thereof), -SO₃H (or salt thereof), -SO₂H (or salt thereof), -NR¹R² (wherein R¹ and R² respectively denote hydrogen atom, or alkyl group having carbon atoms of three or less) or the salt thereof, -NHCO- (amide group), -CHO (aldehyde group), and -PO₂H (salt). Examples of such a hydrophilic monomer may include, e.g., acrylic acid (salt), methacrylic acid (salt), ethylene oxide, vinylsulfonic acid (salt), styrenesulfonic acid (salt), hydroxyethyl methacrylate, acrylamide, etc. Among these, it is particularly preferred to use an acrylic acid (salt) in view of the adhesive property to the material to be coated (particularly, metal).

On the other hand, the "hydrophobic monomer" refers to a monomer which does not have the above-mentioned "hydrophilic group" in the molecule thereof. Such a hydrophobic monomer is not particularly limited as long as it can copolymerize with the above-mentioned hydrophilic monomer. Specific examples of the hydrophobic monomer preferably usable in the present invention may include, e.g., ethylene, propylene, vinyl chloride, styrene, (meth)acrylate derivatives such as ethyl acrylate and methyl methacrylate. Among these, it is particularly preferred to use ethylene in view of easiness in the provision of low-temperature melting property and flexibility suitable for the coating layer according to the present invention.

The method of combining the above-mentioned hydrophilic monomer and hydrophobic monomer in the present invention is not particularly limited as long as they can provide an affect of preventing the adhesion of the body fluid component and a lubricating effect as the coating composition. In view of the balance between these, the content of the hydrophilic monomer (such as acrylic acid (salt)) may preferably be 1 - 50 wt.%, more preferably 3 - 25 wt.% (particularly 6 - 15 wt.%), provided that the total weight of the hydrophobic monomer and the hydrophilic monomer constituting the copolymer (such as ethylene-acrylic acid (salt) copolymer) is 100%. (Method of combining fluorine-containing polymer particulates and non-fluorine-containing polymer)

The method of combining the fluorine-containing polymer particulates and a non-fluorine-containing polymer (such as copolymer or hydrophilic monomer/hydrophobic monomer) constituting the coating composition according to the present invention is not particularly limited as long as the particulates and the non-fluorine-containing polymer are co-present with each other in the composition. In view of the ease of formation of the coating composition, it is particularly preferred to use a method wherein the non-fluorine-containing polymer is formed into particulates and is physically mixed with the fluorine-containing polymer particulates; a method wherein the fluorine-containing polymer particulates are covered with the non-fluorine-containing polymer so as to be formed into composite particulates; etc.

In the case of the former physical mixing method, it is preferred that the above non-fluorine-containing polymer is formed into fine particulates of about 300 µm or less (more preferably about 100 µm or less, particularly about 50 µm or less) by the usual mechanical crushing (at ordinary temperature or under freezing), more preferably by "chemical pulverization", and is mechanically mixed with the above-mentioned fluorine-containing polymer particulates. Herein, the "chemical pulverization" refers to a crushing method utilizing a wet process, i.e., a crushing method wherein a resin is dissolved in a solvent, and then the resin is caused to precipitate by utilizing a change in the solubility of the resin (e.g., based on temperature, pressure, the addition of another solvent, etc.) to thereby obtain a fine powder of the resin.

It is preferred to practice the above-mentioned chemical pulverization of the copolymer by dissolving the non-fluorine-containing copolymer in an appropriate solvent, causing the copolymer to precipitate by use of a change in temperature or pressure, etc., subjecting the resultant product to solid-liquid separation and drying the product, to thereby obtain fine particles in the form of powder.

As the latter method of producing the composite particulates, it is also possible to use, e.g., a method wherein the above-mentioned fluorine-containing polymer particles are used as the "mother" particles and the non-fluorine-containing polymer particulates are used as the "child" particles, and these particles are mechanically formed into a composite by using an ordinary hybridizer apparatus (composite formation by a dry process such as implantation or fine particulates). In view of uniformity in the particle size, it is preferred to obtain composite particulates by utilizing the chemical pulverization method (wet-type crushing method) as described below. When the composite particulates are obtained by utilizing this chemical pulverization method, e.g., the non-fluorine-containing polymer is dissolved in a suitable solvent and the fluorine-containing polymer particulates are mixed with the solution and uniformly dispersed therein, and then the solubility of the non-fluorine-containing polymer is changed on the basis of a change in temperature or pressure, etc., whereby the non-fluorine-containing polymer is caused to precipitate on surfaces of the fluorine-containing polymer particulates as nuclei. After the precipitation of the non-fluorine-containing polymer, the resultant product is subjected to solid-liquid separation and drying, whereby composite particulates can be obtained in the form of powder. In view of the ease of formation of a substantially uniform coating layer, the particle size of the composite particles may preferably be about 300 µm or less, more preferably about 100 µm or less, particularly about 50 µm or less (further 20 µm or less).

In the coating composition according to the present invention, the ratio of the fluorine-containing polymer particulates in the composition may preferably be 5 - 30 wt.%, more preferably 10 - 20 wt.%. The above-mentioned preferred ratio of the fluorine-containing polymer particulates is the same both in the case utilizing the above mechanical mixing (embodiment as shown in Fig. 1), and in the case utilizing the composite fine powder formation (embodiment as shown in Fig. 2). When the mixing ratio of the fluorine-containing polymer is below 5 wt.%, the lubricating property of the coating layer surface has a tendency such that it is liable to be insufficient. On the other hand, when the mixing ratio of fluorine-containing polymer exceeds 30 wt.%, the adhesion of the coating layer to the material to be coated (or the strength of the coating layer) has a tendency such that it is liable to be decreased. Further, in the coating composition of the embodiment to which the non-adhesion to the body fluid component is to be imparted, the antithrombogenic property thereof has a tendency such that it is liable to be decreased.

### (Preferred process for producing composite powder)

In view of the uniformity in the composite powder, the above-mentioned composite powder may preferably be produced, e.g., by the following method. When such a method is applied to the formation of PTFE-EAA composite powder as an embodiment of the above-mentioned composite powder, particularly preferred uniformity in the composite powder (the PTFE is combined with the EAA so as to provide a uniform composite) can be obtained.

Thus, an EAA resin is dissolved in a "good solvent" for the EAA resin, and PTFE fine particles are dispersed in the resultant EAA resin solution. Then, the solubility of the EAA resin in the above-mentioned "good solvent" is decreased to precipitate the EAA resin on each of the PTFE powder particles, thereby to obtain a PTFE-EAA composite powder comprising the PTFE powder particles, the surfaces of which have been coated with the EAA resin (coacervation method).

In such a production process for the PTFE-EAA composite powder using the above-mentioned "good solvent" for the EAA resin, the measure for decreasing the solubility of the EAA resin in the "good solvent" is not particularly limited. For example, as such a measure, it is possible to use any of known measures such as decrease in temperature, addition of a "poor solvent" for the EAA resin, addition of a third substance (with respect to the details of the coacervation method, e.g., Tamotsu Kondo "Microcapsules", page 9 et seq., Kyoritsu Shuppan, in 1985 may be referred to).

When the ratio of the acrylic acid (salt) in the EAA resin is below 15%, in view of ease of manipulation, it is preferred to use an aromatic-type hydrocarbon (such as xylene, toluene and benzene; among these, particularly xylene in view of the safety) as the above-mentioned "good solvent", and to use a measure utilizing a temperature decrease. On the other hand, when the ratio of the acrylic acid (salt) in the EAA resin is 15% or more, in view or the formation of fine particles of the PTFE-EAA composite powder, it is preferred to use a measure of adding a "poor solvent" so as to decrease the solubility of the EAA resin, rather than the above-mentioned measure of using the aromatic hydrocarbon solvent (good solvent) and the temperature decrease.

Specific examples of the good solvent for the abovementioned EAA resin usable in the present invention may include, aqueous ammonia, ammonia derivatives, alkali metal hydroxide aqueous solution. Among these, aqueous ammonia (preferably at a concentration of about 1 - 20%) may particularly preferably be used in view of the adhesion of the thus formed EAA resin to the material to be coated.

As the poor solvent for the above-mentioned EAA resin, it is preferred to use, e.g., an acidic solvent or aqueous solution. Among these, hydrochloric acid (preferably at a concentration of about 1 - 35%) may particularly preferably be used in view of the provision of a minimum amount thereof remaining in the EAA resin.

In the present invention, e.g., the following quantitative ratios may preferably be used, provided that the weight of the EAA resin is used as the standard (ten parts).
Good solvent: 50 - 1000 parts (more preferably 200 - 400 parts)
Poor solvents: 2 - 200 parts (more preferably 50 - 100 parts)
PTFE powder: 0.5 - 3 parts (more preferably 1 - 2 parts)

### (Antithrombogenic property of coating composition)

In view of the non-adhesion of the body fluid component such as antithrombogenic property, etc., the ratio (A/F) of albumin adhesion amount (A) to fibrinogen adhesion amount (F) in the coating composition (or in the coating layer to be described hereinafter) according to the present invention (in the embodiment to which the non-adhesion to the body fluid component is to be imparted) may preferably be about 0.2 or more, more preferably about 0.3 or more (particularly about 0.4 or more). These albumin adhesion amount (A) and fibrinogen adhesion amount (F) may preferably be measured in the following manner.

### 〈Method of measuring adhesion amount〉

Each of I-125 labeled albumin (Human Serum mfd. by ICN Co., about 1 µCi/µg) and I-125 labeled fibrinogen (Human, mfd. by ICN Co., about 1 µCi/µg) is dissolved in 0.1 M-phosphate buffer (pH 7.5) so as to provide a concentration of about 1 mg/ml, and the coating composition according to the present invention is dipped in the resultant aqueous solution while it is left standing therein at 25°C for 15 min. Then, the coating composition is washed with a large amount of 0.1 M-phosphate buffer (pH 7.5), and the amount of the labeled albumin or labeled fibrinogen which has been adsorbed on the coating composition surface is measured by means of a scintillation counter (AUTO WELL GAMMA SYSTEM, ARC-300, mfd. By Aloka Co.).

### (Material to be coated)

The kind of material, shape, size, etc., of the material to be coated are not particularly limited, as long as it can be coated with the coating composition according to the present invention. Specific examples of the kind of the material to be coated may include, e.g., homogeneous or uniform materials, mixtures, complexes or composites, porous materials, which comprise metals, plastics, ceramics, fibers, woody materials, etc. Specific examples of the shape of the material to be coated may include spherical shapes, bar-like shapes, plate-like shapes, cylindrical shapes, hollow cylindrical shapes, indeterminate shapes, mesh shapes, powder shapes, etc.

The material to be coated can be formed into various kinds of shapes depending on the purpose of the use thereof. In the present invention, e.g., the material to be coated may have a shape of medical device, and may have a shape of another device or instrument which is to be inserted into a living body or to be in contact with the surface of a living body (e.g., devices for "piercing" to be inserted into various sites of a living body). The reason for this is that, even if it is a device (such as mesh and gauze) to be used in contact with the surface of a living body, the adhesion of the body fluid component to the device can sometimes cause various kinds of disorder (e.g., accretion between a wound and the device).

In the above-mentioned material to be coated, it is sufficient that at least a surface portion thereof which is to be in contact with the body fluid (or tissue of a living body) is covered with a coating layer based on the coating composition according to the present invention. That is, in the device to be in contact with a living body surface or to be inserted into the living body, it is sufficient that at least a surface portion thereof which is to be in contact with the tissue of a living body or body fluid is covered with the above coating layer.

### (Method of coating surface of material to be coated)

The method of coating the surface of the material to be coated with the coating composition according to the present invention is not particularly limited. It is possible to practice a method wherein the coating composition is attached to the surface of the material to be coated by a spray coating method such as air-spraying, air-less spraying (fluid-pressure atomization method), and electrostatic spraying, electrostatic powder spraying method, etc.; and then the temperature is elevated higher than the softening point of the non-fluorine-containing polymer in the coating composition so as to soften the non-fluorine-containing polymer thereby to effect coating; a fluidization dip coating method wherein the surface of the material to be coated is preheated to a temperature higher than the softening point of the non-fluorine-containing polymer in advance, and the surface is dipped in a fluidizing vessel containing the coating composition according to the present invention so that the coating composition is attached to the surface of the material to be coated and simultaneously the non-fluorine-containing polymer is softened and fluidized so as to form a coating layer; or a flame spray coating method wherein the composition comprising the non-fluorine-containing polymer which has been semi-melted by flame, etc., is jetted to the surface of the material to be coated to effect coating; etc.

When the material to be coated is formed of a metal, any of the above-mentioned coating methods can be practiced. However, when the material to be coated is formed of a polymer material, it is preferred that the softening temperature of the polymer material constituting the material to be coated is sufficiently (preferably by 20°C or more, more preferably by 50°C or more) higher than the softening temperature of the non-fluorine-containing polymer in the coating composition according to the present invention .

When the softening temperature of the polymer material constituting the material to be coated is not sufficiently higher than the softening temperature of the copolymer, it is preferred to effect the coating by a spray coating method or a solvent casting method, etc., by using a solvent capable of dissolving the copolymer.

### (Coating layer)

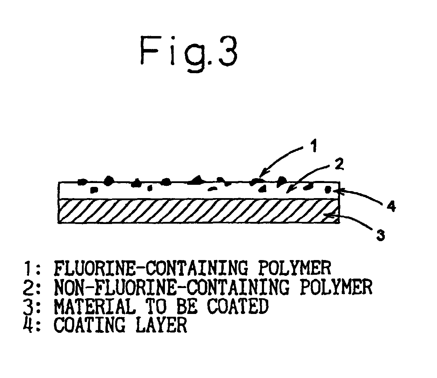

The schematic sectional view of Fig. 3 shows an embodiment or the coating layer which has been formed on the surface of a material to be coated by using the coating composition according to the present invention. Referring to Fig. 3, a coating layer 4 is disposed on the surface of the material to be coated 3. The coating layer 4 comprises particulates 1 comprising a fluorine-containing polymer and a non-fluorine-containing polymer 2, and the particulates 1 are localized in the vicinity of the surface of the layer of the non-fluorine-containing polymer 2.

The above-mentioned coating layer may preferably have a thickness of 5 µm or more, more preferably about 10 - 100 µm (particularly about 20 - 50 µm), in view of the balance between the lubricating property (and/or non-adhesion property to the body fluid such as antithromnbogenic property) and durability (or strength of the coating layer, adhesion property to the material to be coated).

With respect to the coefficient of friction of the coating layer surface, the static friction coefficient (S) thereof may preferably be 1.15 or less, more preferably 1.0 or less (particularly 0.95 or less). On the other hand, the dynamic friction coefficient (D) of the coating layer may preferably be 0.85 or less, more preferably 0.65 or less (particularly 0.50 or less).

### 〈Method of measuring friction coefficient〉

The friction coefficient is measured by means of a surface property measuring apparatus (Tribogear mfd. by Shinto Kagaku Co., Ltd.), by using a SUS 304 ball with a load of 100g at a ball moving speed of about 300 mm/min. (Ratio of fluorine/carbon)

In view of the balance between the lubricating property at the surface of the coating layer and the adhesive property thereof to the material to be coated, with respect to the ratio (F/C) of the fluorine (F) atom and carbon (C) atom in the surface and interior of the coating layer which has been formed on the material to be coated by using the coating composition according to the present invention, the (F/C) ratio (Rs) in the surface may preferably be 0.9 or more, more preferably 1.5 or more (particularly 2.5 or more). Further, the (F/C) ratio (Ri) in the interior (at a depth of 20 µm) may preferably be 2.5 or less, more preferably 2.0 or less (particularly 1.7 or less). The Rs/Ri as the ratio between these (F/C) values may preferably be 1.0 or more, more preferably 1.4 or more (particularly 1.6 or more).

### 〈Method of measuring F/C ratio〉

By use of an Electron Spectroscopy for Chemical Analysis ESCA (ULVAC-PHI Inc., ESCA-SAM 545/548), the (F/C) ratios are measured at five points of a sample and the average of the resultant five values is determined.

The present invention will be described in further detail with reference to the following Examples. However, the scope of the present invention is defined by the Claims and is not limited by these Examples.

### Examples

### Example 1

In a stainless steel tank (capacity 10L, mfd. by Takasago Kakoki Co., Ltd.) equipped with a stirrer and a jacket for adjusting temperature, about 5L of xylene, and about 500g of an ethylene-acrylic acid copolymer (non-fluorine-containing polymer, Primacor 3460, mfd. by Dow Chemical Co., acrylic acid content 9.5 wt.%) were charged under stirring, and were mixed with each other while being heated to about 120°C so that the copolymer was dissolved in the solvent. Then, while the stirring was maintained, the solution temperature was cooled to about 40°C by water cooling so as to cause particulates of the above-mentioned copolymer to precipitate. The thus precipitated particulates were subjected to solid-liquid separation by using a filter press (TFAP-model, mfd. by Tokyo Engineering Kogyo Co., Ltd.), and the resultant recovery cake was dried by a stirring-type vacuum dryer (mfd. by Nihon Kansoki Co., Ltd.) at about 50°C for about 24 hours, and then classified thereby to obtain about 400g of particulates of the ethylene-acrylic acid copolymer having an average particle size of about 40 µm (formation of fine particulates by chemical pulverization).

Then, about 400g of the ethylene-acrylic acid copolymer particulates which had been obtained by the above chemical pulverization were added to about 70g of polytetrafluoroethylene particulates (L-169J, mfd. by Asahi-ICI Fluoro Chemical Co., particle size range 10 - 50 µm, average particle size about 15 µm), and uniformly mixed with each other under stirring, thereby to obtain a coating composition (I).

### Example 2

In the stainless steel tank used in Example 1, about 5L of xylene, about 500g of the ethylene-acrylic acid copolymer used in Example 1, and about 90g of the polytetrafluoroethylene particulates used in Example 1 were mixed with each other under stirring and heated to about 120°C so as to dissolve the copolymer in the solvent. Thereafter, while the stirring was maintained, the solution temperature was cooled to about 40°C by water cooling so as to form composite particles each of which comprised the polytetrafluoroethylene particulate as a core, and the above-mentioned ethylene-acrylic acid copolymer precipitated on the circumference of the nucleus (formation of composite particulates by chemical pulverization).

In the same manner as in Example 1, the thus obtained composite particulates were subjected to solid-liquid separation by using a filter press, and the resultant recovery cake was dried by a stirring-type vacuum dryer at about 50°C, and then classified, thereby to obtain about 550g of composite particulates (coating composition (II)) having an average particle size of about 50 µm.

### Example 3

Composite particulates (coating composition (III)) comprising polytetrafluoroethylene coated with an ethylene-acrylic acid copolymer (Primacor 5980, mfd. by Dow Chemical Co., acrylic acid content about 20 wt.%) were prepared by using the same procedure and conditions as those used in Example 2 except for using such an ethylene-acrylic acid copolymer instead of that used in Example 2 (Primacor 3460, mfd. by Dow Chemical Co., acrylic acid content 9.5 wt.%).

### Example 4

The surface of a stainless steel plate having a size of 6 cm × 10 cm and a thickness of 2 mm was coated with each of the particulates of the ethylene-acrylic acid copolymer prepared in Example 1, and the composite particulates of polytetrafluoroethylene/ethylene-acrylic acid copolymer prepared in Example 2 (polytetrafluoroethylene content: about 15 wt.%; coating composition (II)) by using a fluidization dip coating method in the following manner.

The above-mentioned stainless steel plate was heated in a heating furnace at about 350°C (surface temperature about 290°C) for 10 min. as a pretreatment, and then dipped for about 3 seconds in the fluidizing tank containing the above coating composition, thereby to effect coating of the above-mentioned composition on the stainless steel plate. Then, the resultant stainless steel plate was taken out from the fluidizing tank and the stainless steel plate was heat-treated at about 230°C for about 1 min. as a post treatment. The thickness of each of the thus obtained coating layers was about 40 µm when either of the copolymer particulates and the coating composition (II) was used.

The coefficient of friction of each coating surface was measured by means of a surface property measuring device (Tribogear, mfd. by Shinto Kagaku Co., Ltd.), using a SUS 304 ball with a load of 100g at a ball moving speed of about 300 mm/min. In the case of the ethyleneacrylic acid copolymer coating (single) layer, the static friction coefficient thereof was 1.98, and the dynamic friction coefficient thereof was 0.876. On the other hand, in the case of the polytetrafluoroethylene/ethylene-acrylic acid copolymer coating layer (coating composition (II)), the static friction coefficient thereof was 0.942, and the dynamic friction coefficient thereof was 0.429, whereby the coefficients of friction were markedly decreased.

On the other hand, the ratios (F/C) of the fluorine (F) atom to carbon (C) atom in the surface and interior of both the coating layers were measured by an Electron Spectroscopy for Chemical Analysis ESCA (ULVAC-PHI Inc., ESCA-SAM 545/548). As a result, no fluorine element was detected in the ethylene-acrylic acid copolymer coating layer. On the other hand, in the case of the polytetrafluoroethylene/ethylene-acrylic acid copolymer coating layer, the average of five point measurement (F/C) values was about 2.76 at the surface and was about 1.72 in the interior (depth about 20 µm). From the measurement results of these average (F/C) values, it was presumed that the polytetrafluoroethylene particulates were selectively exposed or localized in the surface portion of the above-mentioned composite particulate coating layer, and that the thus exposed particulates reduced the coefficient of friction markedly.

### Example 5

The Teflon layer with which a Teflon-coated guide wire as a medical guide wire (Boston Scientific Corporation MS/MT/MV Guide Wire, outside diameter: 0.035 inch, length: 145 cm, soft-straight type in the tip portion of 3.5 cm) had been coated was completely peeled by using a cutter so as to expose the metal surface of the wire.

Each of the coating compositions (I) and (II) obtained in Examples 1 and 2 was charged by using the 50KV-electrostatic generator of an electrostatic generator of an electrostatic powder coater (mfd. by Gema Co.), and was applied onto the above-mentioned peeled guide wire for about 4 seconds by spray-coating, and then baking-treated at about 250°C for one minute in a heating furnace, thereby to prepare guide wires which had been coated with the coating compositions (I) and (II), respectively. The thickness of each of the resultant coating layers was 50 - 100 µm.

### Example 6

The fine particulates of the ethylene-acrylic acid copolymer having an average particle size about 40 µm obtained in Example 1 were singly applied onto the guide wire used in Example 5 from which the Teflon coating layer had completely been peeled, by the electrostatic powder coating method used in Example 5, thereby to prepare a guide wire coated with the ethylene-acrylic acid copolymer alone. The thickness of the coating layer was 30 - 100 µm.

### Example 7

The polypropylene surface of a blood vessel indwelling catheter made of polypropylene (Medikit Co., Ltd., Happy-Cath HP-300PP-16, outside diameter 1.98 mm, inside diameter 1.65 mm, effective length 44 mm) was coated with the coating composition (III) obtained in Example 3 in the following manner.

Thus, the coating composition (III) was charged by using the about 50 KV-electrostatic generator used in Example 5, and was applied by spray coating for about 10 seconds onto the polypropylene surface to which an electric conductivity had been imparted by the attachment of water drops by atomizing in advance, whereby a sufficient amount of the coating composition (III) was attached onto the polypropylene surface. Then, the thus obtained polypropylene tube which had been coated with the coating composition (III) was baking-treated in a heating furnace for 5 - 10 min. at a temperature (about 105°C) higher than the melting point (85°C) of the ethylene-acrylic acid copolymer, thereby to prepare a polypropylene tube coated with the coating composition (III). The thickness of the resultant coating layer was 50 - 100 µm.

### Example 8

A part of 16 cm measured from the tip portion (containing the soft-straight portion of 3.5 cm measured from the tip portion) was cut from the guide wire prepared in Example 5 which had been coated with the coating composition (II) according to the present invention, and the antithrombogenic and lubricating properties thereof were evaluated by the following animal experiments.

As the control (comparative) sample, there were used a commercially available Teflon-coated guide wire (Boston Scientific Corporation, MS/MT/MV guide wire) which had similarly been cut at a point 16 cm from the tip portion thereof, and the guide wire prepared in Example 6 which had been coated with only the ethylene-acrylic acid copolymer and had similarly been cut at a point of 16 cm measured from the tip portion thereof.

The reason for the use of each guide wire which had been cut at a point 16 cm from the tip portion thereof was that the experimental animals (rabbits) were small, and the resultant guide wire portion was considered to be easy to be inserted into a blood vessel and to be less liable to damage the inner wall of the blood vessel, since the tip was polished so as to provide a spherically shaped tip, was flexible and was tapered so as to provide a smaller diameter at the tip portion. Both guide wires were sterilized by an ordinary method using ethylene oxide gas.

A rabbit (weight: about 3 kg) was anesthetized by intravenously injecting Nembutal (mfd. by Dainabot Co., Ltd.) in an amount of 25 mg/Kg, and the abdomen thereof was aseptically incised in the median direction so as to expose the inferior vena cava, and the above-mentioned guide wire was directly inserted thereinto from the site which was 1 cm above measured from the iliac vein bifurcation. The length of the inserted portion was about 13 cm, and the tip was positioned in the vicinity of the right atrium. The inserted portion of the guide wire was ligated with 7-0 nylon suture.

The period for the indwelling was 2 - 7 days. After the respective indwelling periods, rapid dehematization was effected by the cannulation into the abdominal aorta, and the inferior vena cava was incised lengthwise, and the blood clot formed on the guide wire surface was examined by visual observation and with an optical microscope (magnification: about 20 ×).

In the case of the guide wire coated with the coating composition according to the present invention (II), substantially no blood clot formation was recognized by visual observation and the microscope observation, even after the indwelling for three days and seven days. The guide wire could be inserted into the blood vessel very smoothly, and a good lubricating property of the guide wire surface was recognised.

On the contrary, in the case of the commercially available Teflon-coated guide wire used as the control sample, the rabbits died after the indwelling for two days. As a result of the autopsy thereof, a large amount of blood clot formation was recognized along the whole length of the guide wire, and it was presumed that the rabbit died because a blood clot markedly decreases the amount of blood flowing in the inferior vena cava. In the case of the Teflon-coated guide wire, the inserting manipulation thereof into the blood vessel was easy, and the lubricating property of the surface thereof was very good.

Further, in the case of the guide wire coated with only the ethylene-acrylic acid copolymer as another control sample, some blood clot formation was recognized in the guide wire surface by visual observation and microscopic observation after the indwelling for three days. In addition, mural or parietal blood clot formation of a medium degree was also recognized on the wall of the inferior vena cava. In the case of this guide wire, the smooth manipulation at the time of the inserting thereof into the blood vessel was difficult due to the frictional resistance with the wall of the blood vessel, and the guide wire had poor surface lubricating property. It was presumed that friction of the wire with the blood vessel wall caused the parietal blood clot formation.

### Example 9

### (Classification of PTFB)

Commercially available Fluon L 169 J (Asahi-ICI Fluoropolymers Co., Ltd.; in the form of fine particulates) was used as PTFE, and was fed into Jet Mill FS-4 mfd. by Seishin Enterprise Co., Ltd., by means of Screw Feeder NX-3000 mfd. by Seishin Enterprise Co., Ltd., at a fixed rate in a throughput of 2 kg/h, and the PTFE particulates which had passed the jet mill were classified by Air-Force Classifier MC-100 mfd. by Seishin Enterprise Co., Ltd., and the resultant fine powder product was collected by a cyclone as a sample. The fine powder which was not collected by the cyclone was collected by a bug filter (equipped with a blower).

The classification conditions used herein were as follows.

### 〈Classification conditions〉

### (Air Classifier MC-100)

Blower: air flow 2.5 m³/min
Clearance ΔH between guide cone end classification cone in MC-100: 30 mm

### (Jet Mill FS-4)

Pusher pressure: 7 kg/cm²
Second air: none
Grinding pressure: 7 kg/cm²
Distribution air: 2 kg/cm²

By the above-mentioned classification operation, there was obtained PTFE fine powder having a particle size range of 1.8 - 27 µm, and was used as a raw material for composite powder. Herein, the PTFE was subjected to wet-type measurement by means of a laser diffraction/scattering-type particle size distribution measuring apparatus LMS-30 mfd. by Seishin Enterprise Co., Ltd., by using IPA (isopropyl alcohol) as a dispersion medium.

### 〈Particle size distribution measurement conditions〉

Dispersion condition: agitation by ultrasonic wave and stirrer
Sample concentration: 616 mV
Shape factor: 1.000
Index or retraction: 1.330 - 0.00i

### Example 10

### (Classification of PTFE-EAA composite powder)

Unclassified PTFE-EAA composite powder which had been obtained in the same manner as in Example 2 was classified by means of a commercially available classifier (High-Volter ST-300S, mfd. By Shin-Tokyo Kikai Co., Ltd., sieve opening: 75 µm), and the portion of the resultant product passing through the sieve was collected by a cyclone, and was used as a painting sample.

At the time of the classification, the sample to be fed into the classification was fed at a constant rate in an amount of 5 kg/h by using Table Feeder FS-J3-S (mfd. by Funken Powtechs Co., Ltd.), and fine powder which had not been collected by the cyclone was collected by a bug filter (equipped with a blower).

By this classification operation, there was obtained PTFE-EAA composite powder having a particle size range of 11 - 76 µm. The particle size was measured by means of a laser diffraction/scattering-type particle size distribution measuring apparatus LMS-30 mfd. by Seishin Enterprise Co., Ltd., by using water as a dispersion medium, and a surfactant as a dispersant.

### 〈Particle size distribution measurement conditions〉

### Dispersion condition: agitation by ultrasonic wave and stirrer

Sample concentration: 1537 mV
Shape factor: 1.000
Index of refraction: 1.330 - 0.00i
Dispersant: sodium dodecyl sulfate
Dispersant concentration: 0.1%.

The thus obtained PTFE-EAA composite powder was applied onto an SS-plate (SS (general-purpose structural rolled steel plate, 50 × 100 × t 1.6 mm) for 1 - 2 seconds by means of an electrostatic coater GX5000 C-1.0 (Mfd. by Nihon Parkerizing Co., Ltd.; air-gun: GX116 (diameter 30 mmφ)) at a voltage of -80 kV, a main air pressure of 1 kg/cm², a sub-air pressure of 1 kg/cm² to effect coating, and the resultant product was heated at 230°C for 3 min, by using a hot-air circulation-type heating furnace, thereby to effect baking. As a result, there could be formed a very thin coating film having an average film thickness (five points of measurement) of 24 µm. When the thus obtained coating film was observed with a High-definition Digital Microscope VH-6300 mfd. by Keyence Corp. (magnification: 85 ×), it was confirmed that the surface was very smooth.

### Example 11

### (Application to needle)

A commercially available needle for heart angiorrhaphy (BV-1 and RB-1, mfd. by Johnson & Johnson Medical Co., Ltd.) was coated with the PTFE-EAA composite powder obtained by the classification in Example 10 (particle size range 11 - 76 µm) by an electrostatic coating method.

This electrostatic coating was effected for 1 - 2 seconds by using an electrostatic caster GX5000 C-1.0 (mfd. by Nihon Parkerizing Co., Ltd.; air-gun: GX 116 (diameter 30 mmφ)) at a voltage of -80 kV, a main air pressure of 1 kg/cm², a sub-air pressure of 1 kg/cm² (formation of coating film). Because the size of the material to be painted (material to be coated) was small, an ion trap was mounted to the above-mentioned air gun and free ions were collected thereby. After the formation of the above coating film, it was heated at 230°C by using a hot-air circulation-type heating furnace for 2 min., thereby to bake the coating film.

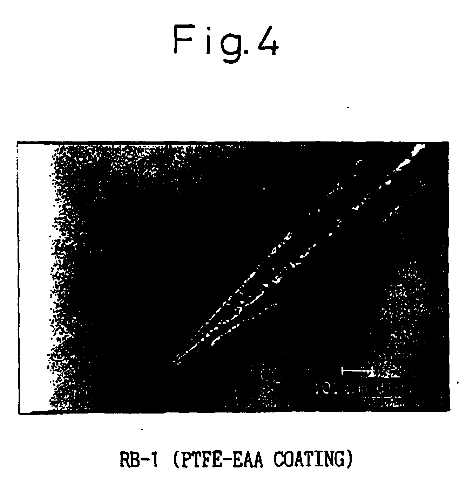

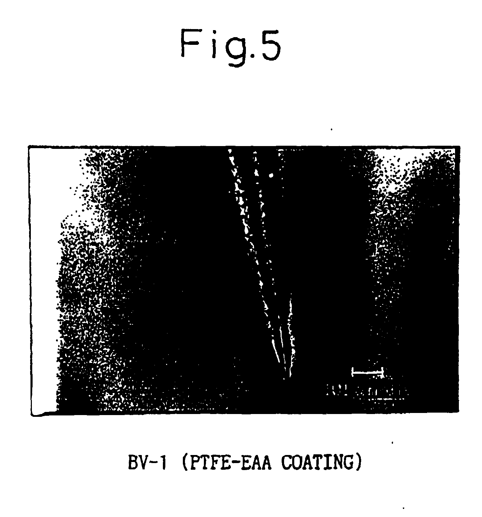

Because the tip portion of the needle as the material to be coated was sharp, electric discharge occurred at the time of the above-mentioned electrostatic coating, and as a result, coating particles were not attached to the tip portion of the needle. Accordingly, in this Example, by utilizing the characteristic of the electrostatic coating, it was possible that the coating was effected while the tip portion of the needle was maintained to be sharp and coating could be effected so as to form a smooth coating film toward the tip portion of the needle (without forming a steep step of the coating layer at the tip potion). The shapes of such a coating was confirmed by the observation with a High-definition Digital Microscope VH-6300 mfd. by Keyence Corp. The images obtained in this observation are shown in Figs. 4 and 5 (magnification: 85 ×).

### Example 12

A coating film was formed on a similar needle under the same coating conditions as in Example 11 (wherein PTFE-EAA composite powder was used) except for using composite powder (PTFE-PET, particle size range 4 - 54 µm) comprising 18 wt. parts of PTFE and 100 wt. parts of modified saturated polyester resin which had been prepared according to Example 1 of the Japanese Patent No. 2034535, instead of the PTFE-EAA composite powder used in Example 11, and the thus obtained coating film was baked by a hot-air circulation-type heating furnace at 320°C for 3 min.

As described above, even when the PTFE-PET composite powder was used, in the same manner as in Example 11 (wherein the PTFE-EAA composite powder was used), coating particles were not attached to the tip portion of the needle, and it was possible that the coating was effected while the tip portion of the needle was maintained to be sharp and coating could be effected so as to form a smooth coating film toward the tip portion of the needle (without forming a steep step of the coating layer at the tip potion).

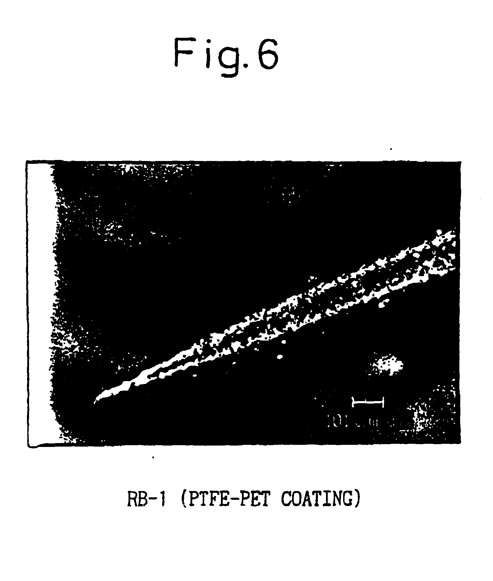

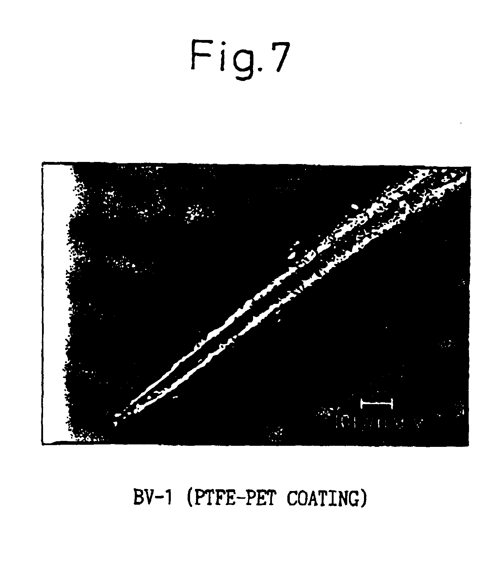

The images obtained in the observation with a Digital Microscope in the same manner as in Example 11 are shown in Figs. 6 and 7 (magnification: 85 ×).

Each of the needles (BV-1 and RB-1) which had been coated with the above-mentioned PTFE-PET coating was firmly grasped at a point of 1/4 to 1/2 of the distance from the root to the needle tip with the tips of a commercially available medical needle-holding device (Hegar's type porte-aiguille, mfd. by Matsuda Ika Kogyo Co., Ltd. equipped with diamond tip, 15 cm) (total ten times, the handle side of the needle-holding device was powerfully pressed by hand).

The above grasped portion was observed with a microscope in the same manner as described above, but substantially no change was recognized in the coating layer.

### Example 13

### (Application to stent)

A commercially available stent (a small spring member for indwelling in a body to be inserted into a balloon-catheter, etc.; trade name: Palmaz Stent, mfd. by Johnson & Johnson Medical Co., Ltd., diameter 2.1 mm × length 15 mm × thickness 0.0055 mm, Cat. No. P 1507) was coated with the PTFE-EAA composite powder obtained in Example 10 (particle size range 11 - 76 µm) by electrostatic coating.

This electrostatic coating was effected for 1 - 2 seconds by using an electrostatic coater GX5000 C-1.0 (mfd. by Nihon Parkerizing Co., Ltd.; air-gun: GX 116 (diameter 30 mmφ)) at a voltage of -80 kV, a main air pressure of 1 kg/cm², a sub-air pressure of 1 kg/cm² (formation of coating film). Because the size of the material to be painted was also small, an ion trap was mounted to the above-mentioned air gun and free ions were collected thereby in the same manner as in Example 11. After the painting, the resultant product was heated at 230°C for 1 min. by using a hot-air circulation-type heating furnace, thereby to bake the coating film.

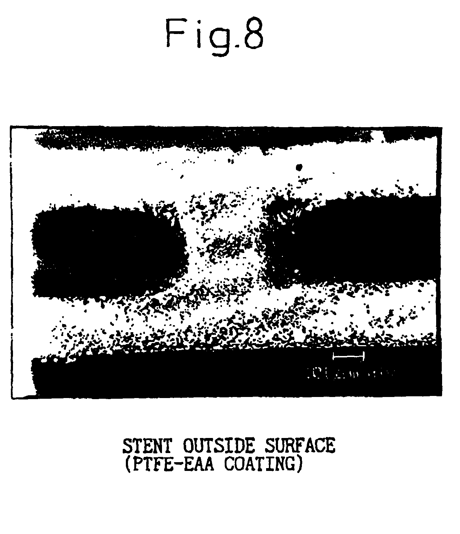

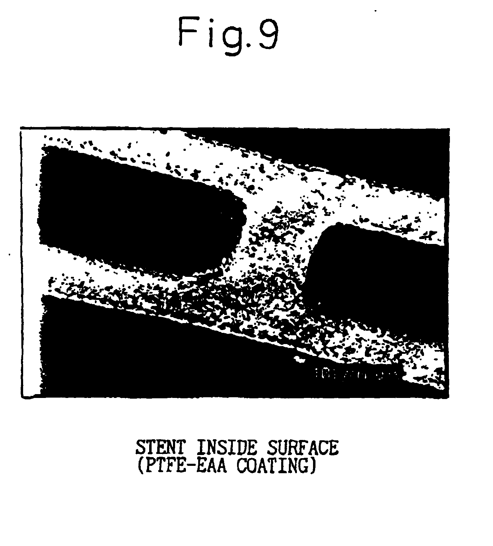

In the case of the stent, it is particularly important to coat the inner side thereof which is to be in direct contact with blood. When the resultant coated sample was observed with a High-definition Digital Microscope VH-6300 mfd. by Keyence Corp., it was confirmed that not only the outside but also the inside of the stent was well coated. The images obtained in the observations are shown in Figs. 8 and 9 (magnification: 85 ×).

According to the present inventors' knowledge, it is presumed that the inside of the stent was also coated, because the PTFE-EAA composite powder used in this Example was very fine and therefore it could enter even the interior of the stent at the time of the coating.

The thus obtained stent which had been coated with the PTFE-EAA coating as described above was expanded by using a commercially available balloon catheter (trade name: OPTA 5 PTA Balloon Catheter. mfd. by Johnson & Johnson Medical Co., Ltd., Cat. No. 530-572 5). After the expansion, the stent had a diameter of 7 mm and a length of 13.5 mm. When the expanded stent was observed with a microscope in the same manner as described above, a change in the coating layer such as peeling or cracking was not recognized.

### Example 14

### (Coating of plastic material)

PTFE-EAA composite powder (for plastic coating) was produced by the following method.

In an agitation dissolution tank having an inner volume of 40 l (equipped with a jacket), 10 l of 5% aqueous ammonia and EAA (trade name: Primacor 5980, mfd. by Dow Chemical Co.) were charged, and thereafter were heated to 95°C and stirred 30 min. while the solution temperature was maintained at 90°C or more. The complete dissolution at the above EAA was confirmed by visual observation, and thereafter the above-mentioned jacket was replaced with water (the water was cooled by using a cooling tower) thereby to effect cooling. After the solution temperature was cooled to 40°C or lower, 10 l of acetone, and 180g of PTFE (trade name: Fluon L 169 J, mfd. by Asahi-ICI Fluoropolymers) were charged and stirred for 10 min., so as to disperse the PTFE in the solution.

Thereafter, 10 l of 10% hydrochloric acid aqueous solution was added to the above-mentioned mixture so as to precipitate the EAA thereby to obtain dispersion of composite powder with PTFE. The dispersion was subjected to solid-liquid separation by means of a filter press, and then was dried by a stirring-type vacuum dryer (equipped with a jacket) at a jacket temperature of 50°C for 24 hours, thereby to obtain PTFE-EAA composite powder.

The thus obtained composite powder was disintegrated by Jet Mill FS-4 mfd. by Seishin Enterprise Co., Ltd., in a throughput of 1 kg/h, thereby to obtain PTFE-EAA composite fine powder having a particle size range of 4 - 64 µm. At this time, the particle size distribution was measured in the same manner as in Example 10 (wet process) by means of a laser diffraction/scattering-type particle size distribution measuring apparatus LMS-30 mfd. by Seishin Enterprise Co., Ltd., by using water as a dispersion medium, and a surfactant as a dispersant.

### Example 15

### (Coating of various plastics)

PTFE-EAA composite fine powder which had been prepared in the same manner as in Example 14 was applied by electrostatic coating onto a material to be coated, including: PP (polypropylene, trade name: Mitsubishi Polypro MA 03, mfd. by Mitsubishi Kagaku Co., Ltd.), HDPE (high-density polyethylene, trade name: Mitsubishi Polyethi-HD, mfd. by Mitsubishi Kagaku Co., Ltd.), PA-6 (polyamide-6, trade name: Grilon A 28 GM, mfd. by Emu-Esu Japan Co., Ltd.), urethane (trade name: Coronate C-4080, mfd. by Nihon Polyurethane Co.), PET (polyethylene terephthalate, trade name: Unitika Polyester Resin NEH-2070, mfd. by Unitika, Co., Ltd.), PVC plate (9 × 5 × thickness 3 mm, polyvinyl chloride, trade name: PVC65, mfd. by Hanyuu Riken Vinyl Co.).

This electrostatic coating was effected for 4 seconds by using an electrostatic coater GX5000 C-1.0 (mfd. by Nikon Parkerizing Co., Ltd.; air-gun: GX 108) at a voltage of -60 kV, a main air pressure or 4 kg/cm², a sub-air pressure of 0.6 kg/cm², thereby to effect coating (formation of coating film). After the formation of the above coating film, the resultant product was heated at 110°C for 5 min. by using a hot-air circulation-type heating furnace, thereby to bake the coating film.

With respect to the above-prepared coated product (six kinds), the adhesion property of the coating film to the material to be coated was evaluated by a "1 mm-cross cut" method. As a result, good adhesion between the coating film and the material to be coated were confirmed except for the PVC. With respect to the details of the above-mentioned "go board section-type tape method", e.g., literature (JIS K 5400) may be referred to.

Further, the above-mentioned PP, HDPE, PA-6, urethane, and PET which had been coated with the PTFE-EAA composite powder were dipped in a large excess of physiological saline solution at room temperature for 24 hours, and thereafter the resultant products were examined with visual observation, whereby the peeling of the above-mentioned PTFE-EAA film was not found.

Separately, the PTFE-EAA film which had been applied onto the above-mentioned PVC was peeled therefrom and formed into a square ample of 45 mm × 45 mm, and was dipped (room temperature) in a large excess of physiological saline solution, and the change in the weight thereof was measured with the elapse or time, so as to determine the water contents of the samples. As a result, the following changes in the water contents were found.

### 〈Change in water content of PTFE-EAA film〉

| Dipping time (hr) | Water content % |
|---|---|
| 0 | 0 |
| 0.5 | 1.6 |
| 1 | 2.5 |
| 2.5 | 3.7 |
| 5 | 5.4 |
| 24 | 11.0 |

### Industrial Applicability

As described hereinabove, according to the present invention, there is provided a coating composition, comprising at least, particulates comprising a fluorine-containing polymer; and a non-fluorine-containing polymer.

The present invention also provides a coating composition comprising at least, particulates comprising a fluorine-containing polymer; and a copolymer comprising a hydrophobic monomer and a hydrophilic monomer.

The present invention further provides a coating method, comprising:
attaching a coating composition comprising at least particulates comprising a fluorine-containing polymer, and a non-fluorine-containing polymer, to the surface of a material to be coated by powder coating; and
heating the non-fluorine-containing polymer to a temperature higher than the softening point thereof, thereby to form a coating layer comprising the coating composition on the surface of the material to be coated.

The present invention further provides a coating method, comprising:
dispersing a coating composition comprising at least particulates comprising a fluorine-containing polymer, and a non-fluorine-containing polymer, in a solvent capable of dissolving the non-fluorine-containing polymer thereby to prepare a coating liquid; and
applying the coating liquid onto the surface of a material to be coated, thereby to form a coating layer comprising the coating composition on the surface of the material to be coated.

The present invention further provides a coated product, comprising a material to be coated and a coating layer covering at least a portion of the material to be coated to be in contact with body fluid; wherein the coating layer comprises at least particulates comprising a fluorine-containing polymer; and a copolymer comprising a hydrophobic monomer and a hydrophilic monomer.

The coating composition having the above-mentioned constitution according to the present invention is capable of forming on various kinds of materials to be coated, a coating which is excellent in the lubricating property (friction-reducing property) and is also excellent in the adhesion property or coating strength to such a base material, etc., on the basis of the above-mentioned combination of the fluorine-containing polymer particulates and non-fluorine-containing polymer (e.g., in a state wherein a portion of the fluorine-containing polymer particulates is exposed to the surface of a coating layer formed on the material to be coated).

Further, the coating composition according to the present invention (in an embodiment to which non-adhesion property to the body fluid has also been imparted) can effectively prevent the adhesion of the body fluid component, which is attached to a portion of the surface of the material to be coated which has been in contact with the body fluid (or has been inserted into a living body) so as to cause a serious problem, or can prevent the blood clot formation based on the above adhesion of the body fluid component, and also facilitates smooth insertion of the material to be coated into the living body. Accordingly, according to the present invention, the lubricating property, and a characteristic capable of reducing damage to the tissue of the living body, which can cause pain, infection, accretion, blood clot formation, etc., are easily imparted to the surface of the material to be coated. Further, the coating composition according to the present invention can cause the resultant coating layer to be strongly bonded to the surface of the material to be coated, and therefore it can reduce the possibility of disorder to a living body which can be caused by the dropping-out of a component constituting the coating layer.

Further, according to the above coating method of the present invention, it is possible to easily coat the contact surface of the material to be coated which is to be in contact with the body fluid, with the above coating composition according to the present invention.

## Claims

1. A coating composition, comprising at least, particulates comprising a fluorine-containing polymer; and a non-fluorine-containing polymer.

2. A coating composition according to claim 1, wherein the non-fluorine-containing polymer is disposed on the circumferences of the particulates.

3. A coating composition according to claim 1, wherein the non-fluorine-containing polymer is a polymer which can be disposed on the circumferences of the particulates by a microencapsulation method.

4. A coating composition according to claim 1, wherein the non-fluorine-containing polymer is a polyester-type polymer or an ethylene-acrylic acid copolymer (EAA).

5. A coating composition according to claim 1 wherein the non-fluorine-containing polymer comprises at least a copolymer comprising a hydrophobic monomer and a hydrophilic monomer.

6. A coating composition according to claim 1 which has a form of composite particulates comprising the fluorine-containing polymer, and the copolymer disposed on the surfaces of the particulates.

7. A coating composition according to claim 6, wherein the fluorine-containing polymer is polytetrafluoroethylene (PTFE), and the hydrophobic monomer constituting the copolymer is ethylene, and the hydrophilic monomer is acrylic acid (salt).

8. A coating method, comprising:
attaching a coating composition comprising at least particulates comprising a fluorine-containing polymer, and a non-fluorine-containing polymer, to the surface of a material to be coated by powder coating; and
heating the non-fluorine-containing polymer to a temperature higher than the softening point thereof, thereby to form a coating layer comprising the coating composition on the surface of the material to be coated.

9. A coating method, comprising:
dispersing a coating composition comprising at least particulates comprising a fluorine-containing polymer, and a non-fluorine-containing polymer, in a solvent capable of dissolving the non-fluorine-containing polymer thereby to prepare a coating liquid; and
applying the coating liquid onto the surface of a material to be coated, thereby to form a coating layer comprising the coating composition on the surface of the material to be coated.

10. A coated product, comprising a material to be coated and a coating layer covering at least a portion of the material to be coated to be in contact with body fluid;
wherein the coating layer comprises at least particulates comprising a fluorine-containing polymer; and a non-fluorine-containing polymer.

11. A coated product according to claim 10, wherein the particulates comprising the fluorine-containing polymer are localized in the vicinity of the surface of the coating layer which is to be in contact with the body fluid.

12. A coated product according to claim 10, wherein the material to be coated has a shape of device to be in contact with the body fluid or to be inserted into the tissue of a living body.

13. A coated product according to claim 12, wherein the device to be in contact with the body fluid or to be inserted into the tissue of a living body has a shape selected from an injection needle, a indwelling needle, a tube, a catheter, a cannula, a guide wire and a stent.

14. A process for producing composite powder, comprising:
dissolving a non-fluorine-containing polymer in a "good solvent" for the non-fluorine-containing polymer;
dispersing particulates comprising a fluorine-containing polymer in the resultant solution of the non-fluorine-containing polymer; and
decreasing the solubility of the non-fluorine-containing polymer in the good solvent so as to precipitate the non-fluorine-containing polymer on the particles comprising the fluorine-containing polymer.

15. A process for producing composite powder according to claim 14, wherein the solubility of the non-fluorine-containing polymer is decreased by adding a poor solvent for the non-fluorine-containing polymer.

16. A process for producing composite powder according to claim 14, wherein the non-fluorine-containing polymer is an EAA resin (ethylene-acrylic acid copolymer).

17. A process for producing composite powder according to claim 14, wherein the fluorine-containing polymer is a PTFE resin (polytetrafluoroethylene).

18. A process for producing composite powder according to claim 14, wherein the good solvent is aqueous ammonia.

19. A process for producing composite powder according to claim 14 wherein the poor solvent is hydrochloric acid.

20. A process for producing composite powder according to claim 14, wherein the particulates comprising the fluorine-containing polymer has an average particle size of 100 µm or less.
